(19) Europäisches Patentamt
European Patent Office
Office européen des brevets

(11) Veröffentlichungsnummer : **0 043 936**
**B1**

(12) # EUROPÄISCHE PATENTSCHRIFT

(45) Veröffentlichungstag der Patentschrift : **05.09.84**

(51) Int. Cl.³ : **C 07 D339/04**, C 07 D495/04

(21) Anmeldenummer : **81104695.2**

(22) Anmeldetag : **19.06.81**

(54) **Verfahren zur Herstellung von Benz-1,2-dithiol-3-thionen.**

(30) Priorität : **12.07.80 DE 3026537**

(43) Veröffentlichungstag der Anmeldung : **20.01.82 Patentblatt 82/03**

(45) Bekanntmachung des Hinweises auf die Patenterteilung : **05.09.84 Patentblatt 84/36**

(84) Benannte Vertragsstaaten : **BE CH DE FR GB LI**

(56) Entgegenhaltungen :
**AT-B- 173 459**
**DE-B- 1 212 560**
**DE-C- 909 097**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Hagen, Helmut, Dr.**
**Max-Slevogt-Strasse 17E**
**D-6710 Frankenthal (DE)**
Erfinder : **Markert, Juergen, Dr.**
**Am Speyerweg 26**
**D-6704 Mutterstadt (DE)**
Erfinder : **Ziegler, Hans, Dr.**
**Am Speyerweg 48**
**D-6704 Mutterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 043 936 B1

**Beschreibung**

Die Erfindung betrifft ein Verfahren zur Herstellung von Benz-1,2-dithiol-3-thionen durch Umsetzung von 2-Halogenbenzylhalogeniden mit Schwefel in Gegenwart von Monoalkylglykoläthern und von als alkanolische Lösungen zugesetzten Alkalialkanolaten, wobei bestimmte Mengenverhältnisse von Schwefel und freiem Alkanol eingehalten werden.

Benz-1,2-dithiol-3-thione sind fungizid wirksam und dienen als Ausgangsprodukte zur Herstellung von Pflanzenschutzmitteln und pharmakologisch wirksamen Stoffen (österreichische Patentschrift 275 965 ; Il Farmaco, Band 26 (1971), Seiten 983 bis 989).

Die US-Patentschrift 3 818 041 beschreibt die Herstellung von aromatischen Trithiocarbonaten und aromatischen, cyclischen Dithiol-Thionen durch Erhitzen von aromatischen Dicarbonsäureanhydriden unter Stickstoff bei 625 bis 800 °C und Umsetzung mit Schwefelkohlenstoff bei dieser Temperatur. Aromatische Nitrodicarbonsäureanhydride werden als Ausgangsstoffe nicht verwendet. Als Ausgangsstoffe werden in den Beispielen nur unsubstituierte bzw. durch Chloratome oder die Carboxygruppe substituierte Phthalsäureanhydride gezeigt.

Die US-Patentschrift 3 040 057 zeigt die Umsetzung von o-Methylbenzolthiolen, die als Substituenten noch eine Alkylgruppe mit 1 bis 4 Kohlenstoffatomen oder eine Phenylgruppe tragen können, mit Schwefel bei Temperaturen von 180 bis 250 °C. Es wird darauf hingewiesen, daß Ausgangsstoffe mit Alkylsubstituenten mit mehr als 4 Kohlenstoffatomen ungeeignet sind, da sie mit Schwefel reagieren, teerige Nebenprodukte bilden und die Ausbeute an Endstoff bis auf unwesentliche Mengen verringern können ; die Nebenstoffe behindern darüber hinaus die Abtrennung des Endstoffs erheblich.

Die britische Patentschrift 1 391 679 lehrt die Umsetzung von o-Halogenbenzylhalogeniden und Derivaten davon mit weniger als 3 bis gegen etwa 6 Mol Schwefel zu 1 Mol Ausgangschlorid, vorzugsweise in Dimethylformamid (DMF) oder homologen Formamiden und Acetamiden, oder in einem Alkohol in Gegenwart von Alkalialkoholat, wobei in der Regel als Alkohol der schon im Alkalialkoholat gebundene Alkohol verwendet wird. So werden (Beispiel 2) aus 120,7 g o-Chlorbenzylchlorid und 120 g Schwefel in DMF 42 g Benzo-1,2-dithiol-3-thion enthalten. Die Kombination Methanol + Natriummethoxylat wird lediglich durch Beispiel 5 für die Herstellung von Benzo-[1,2,d ; 5,6,d]-tri-(1,2-dithiol-3-thion) belegt, wo der dreifache Ringschluss zu einer Ausbeute an rohem Endstoff von lediglich 4,5 % der Theorie führt.

Die deutsche Offenlegungsschrift 24 60 783 beschreibt ein Verfahren zur Herstellung von 5-Nitrobenz-1,2-dithiol-3-thion durch Umsetzung von 2-Halogen-5-nitrobenzylhalogeniden mit elementarem Schwefel in Gegenwart einer basischen Verbindung bei einer Temperatur von 20 bis 100 °C. Ihre Lehre bezieht sich nicht allgemein auf die Klasse der Benz-1,2-dithiol-3-thionverbindungen, sondern ausdrücklich auf eine einzige, durch eine Nitrogruppe in 5-Stellung substituierte Verbindung. Es werden zwar eine große Zahl von organischen Lösungsmitteln, darunter auch Methylglykoläther und Alkanole, angegeben, in den Beispielen wird aber stets Methanol verwendet. Es wird mehrfach darauf hingewiesen, daß die Verwendung von organischen Lösungsmitteln nicht obligatorisch, sondern nur zweckmäßig ist. Als bevorzugte basische Verbindungen werden tertiäre Amine, Erdalkali- und Alkaliverbindungen, vorzugsweise Alkoholate, angegeben. Es wird aber hervorgehoben, daß Methanol als Lösungsmittel zusammen mit Trialkylaminen mit 1 bis 4 Kohlenstoffatomen als basischen Verbindungen bevorzugt ist.

Es wurde nun gefunden, daß man Benz-1,2-dithiol-3-thione der Formel

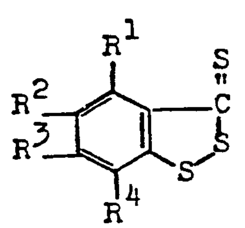

(I)

worin $R^1$ ein Wasserstoffatom bedeutet, $R^2$ ein Wasserstoffatom, ein Halogenatom, einen Alkylrest mit 1 bis 6 Kohlenstoffatomen, einen Benzoylrest, Naphthoylrest oder den Rest

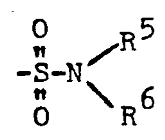

bezeichnet, worin $R^5$ und $R^6$ gleich oder verschieden sein können und jeweils für ein Wasserstoffatom, einen Alkylrest mit 1 bis 6 Kohlenstoffatomen, einen Cycloalkylrest mit 5 bis 8 Kohlenstoffatomen, einen Aralkylrest oder Alkylarylrest mit jeweils 7 bis 12 Kohlenstoffatomen, einen Phenylrest, einen Naphthylrest stehen, darüber hinaus $R^5$ und $R^6$ zusammen mit dem benachbarten Stickstoffatom auch Glieder eines Ringes, der außer dem Stickstoffatom noch ein Sauerstoffatom enthalten kann, bedeuten, $R^3$ und $R^4$

gleich oder verschieden sein können und jeweils ein Wasserstoffatom oder einen Alkylrest mit 1 bis 6 Kohlenstoffatomen bedeuten, zwei benachbarte Reste unter den Resten $R^1$, $R^2$, $R^3$ und $R^4$ auch jeweils zusammen einen anellierten Benzolkern, anellierten Naphthalinkern oder anellierten Pyridinkern bedeuten können, durch Umsetzung von 2-Halogenbenzylhalogeniden mit elementarem Schwefel und in Gegenwart einer basischen Verbindung und eines organischen Lösungsmittels vorteilhaft erhält, wenn man 2-Halogenbenzylhalogenide der Formel

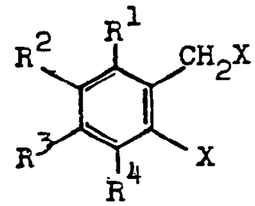

(II)

worin $R^1$, $R^2$, $R^3$ und $R^4$ die vorgenannte Bedeutung besitzen und die einzelnen Reste X gleich oder verschieden sein können und jeweils ein Chloratom oder ein Bromatom bezeichnen, mit Schwefel im Verhältnis von 2,5 bis 3,5 Grammatom Schwefel zu 1 Mol Ausgangsstoff II bei einer Temperatur von 50 bis 200 °C in Gegenwart von Monoalkylglykoläthern als Lösungsmittel und einem in Gestalt einer alkanolischen Lösung zugesetztem Alkalialkanolat als basischer Verbindung umsetzt, wobei man die Gesamtmenge an freiem Alkanol während der Reaktion auf ein Verhältnis von 0 bis 30 Gewichtsprozent Alkanol, bezogen auf Monoalkylglykoläther, einstellt.

Die Umsetzung kann für den Fall der Verwendung von 2-Chlorbenzylchlorid und Natriummethylat durch die folgenden Formeln wiedergegeben werden :

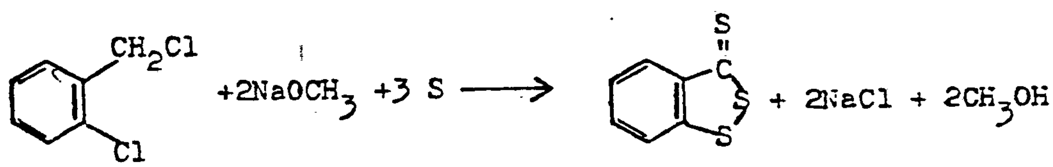

Im Vergleich zu dem aus der britischen Patentschrift 1 391 679 bekannten Verfahren liefert das Verfahren nach der Erfindung Benz-1,2-dithiol-3-thione in besserer Ausbeute und Reinheit. Diese vorteilhaften Ergebnisse sind überraschend.

Die Ausgangsstoffe II können aus den entsprechenden 2-Halogenmethylverbindungen durch Photohalogenierung erhalten werden. Die 3-Halogenmethyl-4-halogenbenzolsulfonamide II kann man durch Umsetzung von 3-Chlormethyl-4-chlorbenzolsulfonsäurechlorid mit einer äquimolaren Menge des entsprechenden Amins in einem inerten Lösungsmittel, z. B. Benzol, in Gegenwart einer Base, z. B. tertiäre Amine wie Triäthylamin, erhalten.

Der Ausgangsstoff II wird mit Schwefel in einem Verhältnis von 2,5 bis 3,5, vorzugsweise 3 bis 3,2 Grammatom Schwefel je Mol Ausgangsstoff II umgesetzt. Wenn im Ausgangsstoff II und dementsprechend im Endstoff I Halogenatom bedeutet, so handelt es sich vorzugsweise um $R^2$ ein Bromatom oder zweckmäßiger ein Chloratom. Ferner ist ein von $R^5$ und $R^6$ zusammen mit dem benachbarten Stickstoffatom gebildeten Ring, der außer dem Stickstoffatom noch ein Sauerstoffatom enthalten kann, vorzugsweise 5- oder 6-gliedrig.

Als Ausgangsstoffe II sind z. B. folgende 2-Halogenbenzylhalogenide geeignet : 2-Chlorbenzylchlorid ; in 5-Stellung, 6-Stellung und/oder 7-Stellung einfach, gleich oder unterschiedlich zweifach oder dreifach durch die Methyl-, Athyl-, Propyl-, Isopropyl-, Butyl-, Isobutyl-, sek.-Butyl-, tert.-Butyl-gruppe substituierte 2-Chlorbenzoylchloride ; homologe 5-Benzoyl-, 5-Naphthoyl-, 5-Chlor-, 5-Brom-2-Chlorbenzoylchloride ; entsprechende an den Stickstoffatomen unsubstituierte oder einfach oder gleich oder unterschiedlich zweifach durch die Methyl-, Äthyl-, Propyl-, Isopropyl-, Butyl-, Isobutyl-, sek.-Butyl-, tert.-Butyl-, Cyclohexyl-, Benzyl-, Phenyl-gruppe substituierte 5-Sulfonamido-2-chlorbenzoyl-chloride ; unsubstituiertes und entsprechend mit den vorgenannten Gruppen substituiertes und entsprechend mit den vorgenannten Gruppen substituiertes 1-Chlor-2-chlormethyl-, 2-Chlor-3-chlormethyl-, 3-Chlor-4-chlormethyl-naphthalin, 1-Chlor-2-chlormethyl-, 2-Chlor-3-chlormethyl-, 3-Chlor-4-chlormethyl-anthracen, 1-Chlor-2-chlormethyl-, 2-Chlor-3-chlormethyl-, 3-Chlor-4-chlormethyl-phenanthren ; entsprechende durch Chlor und die Chlormethylgruppe in 5,6-, 6,7- und 7,8-Stellung substuierte Chinoline ; entsprechende 2-Brommethyl-1-bromide, 2-Chlormethyl-1-bromide und 2-Brommethyl-1-chloride.

Die Umsetzung wird bei einer Temperatur von 50 bis 200 °C, vorzugsweise 90 bis 120 °C, insbesondere 98 bis 110 °C, drucklos oder unter Druck, kontinuierlich oder diskontinuierlich durchgeführt. Als Lösungsmittel werden vorzugsweise die Monoalkyläther des Äthylenglykols mit einer Alkylgruppe von 1 bis 6 Kohlenstoffatomen, vorteilhaft der Methyl-, Äthyl-, Propyl-, Isopropyl-, Butyl-, Isobutyl-, sek.-Butyl-, tert.-Butyl-monoäther des Äthylenglykols, verwendet. Zweckmäßig verwendet man das Lösungsmittel in einer Menge von 400 bis 10 000 Gewichtsprozent, vorzugsweise von 1 000 bis 2 000 Gewichtsprozent, bezogen auf Ausgangsstoff II. Weiterhin werden bei der Umsetzung alkanolische

Alkalialkoholatlösungen, zweckmäßig solche des Natriumalkanolats oder Kaliumalkanolats, verwendet. Als Alkanolat und/oder freies Alkanol kommen bevorzugt solche mit 1 bis 6 Kohlenstoffatomen in Frage, insbesondere Methylalkohol, Äthylalkohol, Propyl-, Isopropyl-, Butyl-, sek.-Butyl-, Isobutyl-, tert.-Butylalkohol und/oder Alkalimethylat, -äthylat, -propylat, -isopropylat, -butylat, -isobutylat, -sek.-butylat, -tert.-butylat, in Betracht. Die Lösungen sind zweckmäßig 5- bis 40-, vorzugsweise 10- bis 20-gewichtsprozentig. Während der Reaktion entwickelt sich durch die Umsetzung des Alkanolats mit dem freiwerdenden Halogenwasserstoff eine weitere Menge an freiem Alkanol, so daß während der Reaktion gleichzeitig Anteile von als Lösungsmittel des Alkanolats verwendeten und aus dem Alkanolat gebildetem Alkanol vorhanden sind. Man kann nun ein Verhältnis (Lösungsmittelverhältnis) von 0 bis 30, vorteilhaft 0,1 bis 25, insbesondere 0,1 bis 10 Gewichtsprozent freies Alkanol, bezogen auf den Monoalkylglykoläther, derart einstellen, daß man vor Beginn der Reaktion eine so hochkonzentrierte Alkanolatlösung vorlegt, daß auch nach Bildung von weiterem Alkanol aus dem Alkanolat die vorgenannte Maximalmenge an Alkanol nicht überschritten wird. Man kann aber auch höhere Mengen an Alkanol verwenden und während der Reaktion durch gleichzeitiges Abdestillieren entsprechender Alkanolmengen das erfindungsgemäße Lösungsmittelverhältnis einstellen. Das erfindungsgemäße Lösungsmittelverhältnis kann von Anfang an bestehen oder erst während der Reaktion einstellt werden ; bevorzugt ist das erfindungsgemäße Verhältnis an Alkanol nach 0 bis 2 Stunden ab Reaktionsbeginn eingestellt und wird dann bis zum Reaktionsende innerhalb der vorgenannten Intervallgrenzen eingehalten. Bei der Berechnung des Verhältnisses wird hier unter Alkanol stets das freie (vorgelegte und gebildete) Alkanol verstanden, das im Alkalialkoholat gebundene Alkanol bleibt für die Berechnung außer Betracht. Zwar können freies Alkanol und im Alkalialkanolat gebundenes Alkanol unterschiedlich sein, in der Regel wählt man aber dasselbe Alkanol. Das Alkanolat kann in stöchiometrischer Menge oder im Überschuß, zweckmäßig in einer Menge von mindestens 1, vorzugsweise 1 bis 4, insbesondere 1,8 bis 2,2 Mol Alkalialkanolat, bezogen auf Ausgangsstoff II, umgesetzt werden.

Die Reaktion kann wie folgt durchgeführt werden : Ein Gemisch von Ausgangsstoff II, Schwefel, alkanolischer Alkalialkanolatlösung und Monoalkylglykoläther wird während 5 bis 49 Stunden, vorzugsweise 10 bis 20 Stunden, bei der Reaktionstemperatur gehalten. Vorteilhaft erhitzt man ein ungefähr stöchiometrisches Gemisch aus Ausgangsstoff II und Schwefel im Alkylglykoläther für eine halbe Stunde auf 100 bis 110 °C, gibt dann das Alkanolat zu und hält das Reaktionsgemisch noch 10 bis 48 Stunden bei der Reaktionstemperatur. Nun wird aus dem Gemisch der Endstoff in üblicher Weise, z. B. durch Filtration oder Destillation und Waschen mit Wasser, isoliert.

Die nach dem Verfahren der Erfindung herstellbaren Verbindungen I sind Wirkstoffe und wertvolle Ausgangsstoffe für die Herstellung von Farbstoffen, Pharmazeutika, Fungiziden, Bakteriziden und Insektiziden, die insbesondere im Pflanzenschutz und zum Schutz von technischen Werkstoffen, wie z. B. Holz, Verwendung finden. Bezüglich der Verwendung wird auf die vorgenannten Veröffentlichungen verwiesen.

Die in den Beispielen angegebenen Teile bedeuten Gewichtsteile.

## Beispiel 1

1 610 Teile 2-Chlorbenzylchlorid werden mit 960 Teilen Schwefel in 5 000 Teilen Methylglykol 30 Minuten bei 100 °C gerührt. Anschließend werden 3 600 Teile 30-gewichtsprozentige, methanolische Natriummethylatlösung zugegeben, während gleichzeitig Methanol über eine Kolonne abdestilliert wird. Das Reaktionsgemisch wird 48 Stunden bei 100 °C gerührt. Während der Reaktion wird ein Verhältnis von 0 bis 5 Gewichtsprozent freies Alkanol, bezogen auf Methylglykol, eingehalten. Man destilliert 60 % des Lösungsmittels ab und kristallisiert den im Rückstand verbleibenden Endstoff um (Aceton). Man erhält 1 435 Teile Benz-1,2-dithiol-3-thion, entsprechend 78 % der Theorie. Der Schmelzpunkt beträgt 92 bis 93 °C.

## Beispiel 2

322 Teile 2-Chlorbenzylchlorid werden mit 192 Teilen Schwefel in 5 000 Teilen Methylglykol 30 Minuten bei 100 °C gerührt. Anschließend werden 488 Teile Kalium-tert.-butylat in tert. Butanol portionsweise zugegeben. Das Reaktionsgemisch wird 48 Stunden bei 100 °C gerührt. Während der Reaktion wird ein Verhältnis von 8 Gewichtsprozent freies Alkanol, bezogen auf Monoalkylglykoläther, eingehalten. Man nimmt die Reaktionslösung in Dichlormethan auf, wäscht dreimal mit Wasser und engt die organische Phase ein. Nach Umkristallisation aus Aceton werden 290 Teile Benz-1,2-dithiol-3-thion (79 % der Theorie) erhalten. Fp 92 bis 93 °C.

## Beispiel 3

32,2 Teile 2-Chlorbenzylchlorid werden mit 19,2 Teilen Schwefel in 500 Teilen Butylglykol auf 100 °C erwärmt und bei dieser Temperatur mit 72 Teilen 30-gewichtsprozentiger Natriummethylatlösung versetzt. Das Reaktionsgemisch wird 48 Stunden bei 100 °C gerührt. Während der Reaktion wird ein Verhältnis von 9 Gewichtsprozent freies Alkanol, bezogen auf Monoalkylglykoläther, eingehalten. Die

Aufarbeitung erfolgt analog Beispiel 2. Man erhält 25 Teile Benz-1,2-dithiol-3-thion (68 % der Theorie) vom Fp 92 bis 93 °C.

Beispiel 4

32,2 Teile 2-Chlorbenzylchlorid werden mit 19,2 Teilen Schwefel in 500 Teilen Methylglykol auf 100 °C erwärmt und bei dieser Temperatur mit 63 Teilen Natriumstearylalkoholat in Stearylalkohol versetzt. Das Reaktionsgemisch wird 48 Stunden bei 100 °C gerührt und analog Beispiel 2 aufgearbeitet. Während der Reaktion wird ein Verhältnis von 12 Gewichtsprozent freies Alkanol, bezogen auf Monoalkylglykoläther, eingehalten. Der ölige Rückstand wird über Kieselgel mit Ligroin/Toluol chromatographisch aufgearbeitet. Man erhält 20 Teile (54 % der Theorie) Benz-1,2-dithiol-3-thion vom Fp 92 bis 93 °C.

Beispiel 5

195,5 Teile 2,5-Dichlorbenzylchlorid werden mit 96 Teilen Schwefel in 2 000 Teilen Methylglykol 15 Minuten bei 100 °C gerührt. Anschließend werden 360 Teile 30-gewichtsprozentige methanolische Natriummethylatlösung zugegeben. Das Reaktionsgemisch wird 24 Stunden bei 100 °C gerührt. Während der Reaktion wird ein Verhältnis von 15 Gewichtsprozent freies Alkanol, bezogen auf Methylglykol, eingehalten. Danach wird das Lösungsmittel im Vakuum entfernt, der Rückstand mit Wasser gewaschen und das 5-Chlorbenz-1,2-dithiol-3-thion mit Äther auskristallisiert. Man erhält 180 Teile 5-Chlorbenz-1,2-dithiol-3-thion (82 % der Theorie) vom Schmelzpunkt 130 °C.

Beispiel 6

51,1 Teile 1-Chlor-2-bromethylnaphthalin werden mit 19,2 Teilen Schwefel in 800 Teilen Methylglykol 30 Minuten bei 110 °C gerührt. Anschließend werden 21,6 Teile 30-gewichtsprozentige methanolische Natriummethylatlösung zugegeben und das Reaktionsgemisch 18 Stunden bei 110 bis 115 °C gerührt. Während der Reaktion wird ein Verhältnis von 5 Gewichtsprozent freies Alkanol, bezogen auf Methylglykol, eingehalten. Nach Erkalten wird der ausgefallene Niederschlag abgesauft und mit Wasser gewaschen. Man erhält 41 Teile (88 % der Theorie) Naphto-[1,2]-1,2-dithiol-3-thion vom Schmelzpunkt 166 °C.

Beispiel 7

530 Teile 3-Chlormethyl-4-chlorbenzophenon werden mit 192 Teilen Schwefel in 1 500 Teilen Methylglykol auf 100 °C erwärmt. Anschließend werden 720 Teile 30-gewichtsprozentige methanolische Natriummethylatlösung zugegeben, während gleichzeitig das überschüssige Methanol über eine Kolonne abdestilliert wird. Das Reaktionsgemisch wird 24 Stunden bei 100 °C gerührt. Während der Reaktion wird ein Verhältnis von 5 Gewichtsprozent Methanol, bezogen auf Methylglykol, eingehalten. Nach Erkalten wird der ausgefallene Nieschlag abgetrennt, mit Wasser gewaschen, getrocknet und mit Diäthyläther behandelt. Man erhält 260 Teile (45 % der Theorie) 5-Benzoyl-benz-1,2-dithiol-3-thion vom Schmelzpunkt 162 bis 164 °C.

Beispiel 8

268 Teile N,N-Dimethyl-3-chlormethyl-4-chlorbenzolsulfonamid werden mit 96 Teilen Schwefel in 1 000 Teilen Methylglykol auf 100 °C erwärmt. Anschließend werden 360 Teile 30-gewichtsprozentige mechanolische Natriummethylatlösung innerhalb von 2 Stunden zugegeben, während gleichzeitig Methanol über eine Kolonne abdestilliert wird. Das Reaktionsgemisch wird 48 Stunden bei 100 °C gerührt. Während der Reaktion wird ein Verhältnis von 5 Gewichtsprozent freies Methanol, bezogen auf Methylglykol, eingehalten. Die Reaktionslösung wird abgekühlt, der ausgefallene Feststoff abgesaugt und mit Wasser und mit Äther gewaschen. Man erhält 215 Teile 5-N,N-Dimethylsulfonamido-benz-1,2-dithiol-3-thion (74 % der Theorie) vom Schmelzpunkt 185 °C.

Beispiel 9

76,2 Teile N-Methyl-3-chlormethyl-4-chlorbenzolsulfonamid werden mit 28,8 Teilen Schwefel in 500 Teilen Äthylglykol 30 Minuten lang auf 100 °C erwärmt. Anschließend werden 108 Teile 30-gewichtsprozentige Natriummethylatlösung zugegeben. Das Reaktionsgemisch wird 24 Stunden bei 90 °C gerührt. Während der Reaktion wird ein Verhältnis von 13 Gewichtsprozent freies Alkanol, bezogen auf Monoalkylglykoläther, eingehalten. Nach Erkalten wird filtriert und die Reaktionslösung im Vakuum eingeengt. Der Rückstand wird mit Wasser gewasche,ß getrocknet und mit Dichlormethan behandelt. Man erhält 46 Teile 5-N-Methylsulfonamido-benz-1,2-dithiol-3-thion (55 % der Theorie) vom Schmelzpunkt 171 °C.

Beispiele 10 bis 17

Analog Beispiel 8 werden die in der Tabelle gezeigten Umsetzungen durchgeführt.

Tabelle

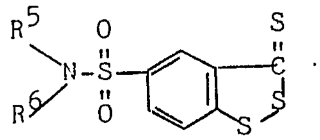

| Beispiel | | Ausbeute in % der Theorie | Fp in °C |
|---|---|---|---|
| 10 | $R^5$, $R^6$ = iso-$C_3H_7$ | 55 | 136 |
| 11 | $R^5$, $R^6$ = 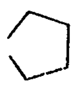 | 60 | 160 - 162 |
| 12 | $R^5$ = H, $R^6$ = n-$C_4H_9$ | 57 | 150 - 155 |
| 13 | $R^5$ = $CH_3$, $R^6$ = $C_6H_5$ | 45 | 148 |
| 14 | $R^5$ = H, $R^6$ = $C_2H_5$ | 63 | 161 |
| 15 | $R^5$, $R^6$ = 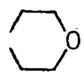 | 58 | 180 |
| 16 | $R^5$ = H, $R^6$ = iso-$C_3H_7$ | 50 | 171 |

Beispiel 17

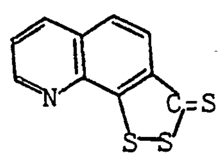

42 Teile 7-Chlormethyl-8-chlorchinolin werden analog Beispiel 1 mit 19,2 Teilen Schwefel und 72 Teilen 30-gewichtsprozentiger methanolischer Natriummethylatlösung umgesetzt. Während der Reaktion wird ein Verhältnis von 8 Gewichtsprozent freies Alkanol, bezogen auf Monoalkylglykoläther, eingehalten. Man erhält 18 Teile Chinolino-[8,7-d]-1,2-dithiol-3-thion (40 % der Theorie) vom Fp (Zers.) 300 °C.

**Anspruch**

Verfahren zur Herstellung von Benz-1,2-dithiol-3-thionen der Formel

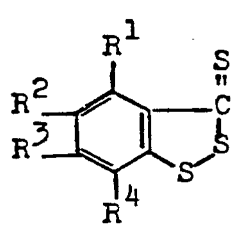

(I)

worin $R^1$ ein Wasserstoffatom bedeutet, $R^2$ ein Wasserstoffatom, ein Halogenatom, einen Alkylrest mit 1 bis 6 Kohlenstoffatomen, einen Benzoylrest, Naphthoylrest oder den Rest

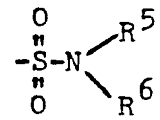

bezeichnet, worin $R^5$ und $R^6$ gleich oder verschieden sein können und jeweils für ein Wasserstoffatom, einen Alkylrest mit 1 bis 6 Kohlenstoffatomen, einen Cycloalkylrest mit 5 bis 8 Kohlenstoffatomen, einen Aralkylrest oder Alkylarylrest mit jeweils 7 bis 12 Kohlenstoffatomen, einen Phenylrest, einen Naphthylrest stehen, darüber hinaus $R^5$ und $R^6$ zusammen mit dem benachbarten Stickstoffatom auch Glieder eines Ringes, der außer dem Stickstoffatom noch ein Sauerstoffatom enthalten kann, bedeuten, $R^3$ und $R^4$ gleich oder verschieden sein können und jeweils ein Wasserstoffatom oder einen Alkylrest mit 1 bis 6 Kohlenstoffatomen bedeuten, zwei benachbarte Reste unter den Resten $R^1$, $R^2$, $R^3$ und $R^4$ auch jeweils zusammen einen anellierten Benzolkern, anellierten Naphthalinkern oder anellierten Pyridinkern bedeuten können, durch Umsetzung von 2-Halogenbenzylhalogeniden mit elementarem Schwefel in Gegenwart einer basischen Verbindung und eines organischen Lösungsmittels, dadurch gekennzeichnet, daß man 2-Halogenbenzylhalogenide der Formel

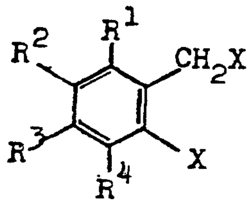

(II)

worin $R^1$, $R^2$, $R^3$ und $R^4$ die vorgenannte Bedeutung besitzen und die einzelnen Reste X gleich oder verschieden sein können und jeweils ein Chloratom oder ein Bromatom bezeichnen, mit Schwefel im Verhältnis von 2,5 bis 3,5 Grammatom Schwefel zu 1 Mol Ausgangsstoff II bei einer Temperatur von 50 bis 200 °C in Gegenwart von Monoalkylglykoläthern als Lösungsmittel und einem in Gestalt einer alkanolischen Lösung zugesetztem Alkalialkanolat als basischer Verbindung umsetzt, wobei man die Gesamtmenge an freiem Alkanol während der Reaktion auf ein Verhältnis von 0 bis 30 Gewichtsprozent Alkanol, bezogen auf Monoalkylglykoläther, einstellt.

**Claim**

A process for the preparation of a benz-1,2-dithiol-3-thione of the formula

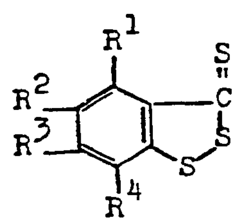

(I)

where $R^1$ is hydrogen, $R^2$ is hydrogen, halogen, alkyl of 1 to 6 carbon atoms, benzoyl, naphtoyl or

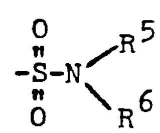

where $R^5$ and $R^6$ may be identical or different and each denote hydrogen, alkyl of 1 to 6 carbon atoms, cycloalkyl of 5 to 8 carbon atoms, aralkyl or alkylaryl each having 7 to 12 carbon atoms, phenyl or naphthyl, or $R^5$ and $R^6$ together with the adjacent nitrogen atom are members of a ring which may contain an oxygen atom in addition to the nitrogen atom, and $R^3$ and $R^4$ may be identical or different and each denote hydrogen or alkyl of 1 to 6 carbon atoms, it being possible for adjacent pairs of the radicals $R^1$, $R^2$, $R^3$ and $R^4$ to denote a fused benzene, naphthalene or pyridine nucleus, by reacting a 2-halobenzylhalide with elementary sulphur in the presence of a basic compound and an organic solvent, wherein a 2-halobenzylhalide of the formula

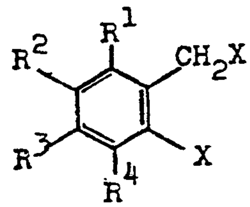

(II)

where $R^1$, $R^2$, $R^3$ and $R^4$ have the above meanings and the individual X's may be identical or different and each denote chlorine or bromine is reacted with sulphur in a ratio of 2.5 to 3.5 gram atom of sulphur per mole of starting material II at a température of 50 to 200 °C in the presence of a monoalkyl glycol ether as solvent and of an alkali metal alkanolate, as basic compound added in the form of a solution in alkanol, the total amount of free alkanol during the reaction being adjusted to 0 to 30 % by weight, based on the monoalkyl glycol ether.

**Revendication**

Procédé de préparation de benzo-1,2-dithiol-3-thiones de la formule

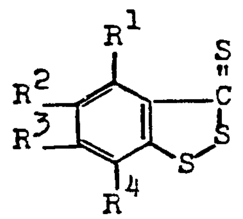

(I)

dans laquelle $R^1$ désigne un atome d'hydrogène ; $R^2$ un atome d'hydrogène ou d'halogène, un radical alkyle en $C_1$ à $C_6$ ou un groupe benzoyle, naphtyle ou

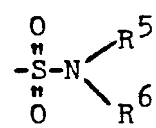

où $R^5$ et $R^6$, qui peuvent être identiques ou différents, désignent chacun un atome d'hydrogène, un radical alkyle en $C_1$ à $C_6$ ou un groupe cycloalkyle en $C_5$ à $C_8$, aralkyle ou alkylaryle en $C_7$ à $C_{12}$, phényle ou naphtyle, ou forment avec l'atome d'azote adjacent des chaînons d'un noyau cyclique qui, à côté de l'atome d'azote, peut comprendre un atome d'oxygène ; $R^3$ et $R^4$, qui peuvent être identiques ou différents, représentent chacun un atome d'hydrogène ou un radical alkyle en $C_1$ à $C_6$, deux substituants voisins parmi les substituants $R^1$, $R^2$, $R^3$ et $R^4$ pouvant en outre former un noyau benzène ou naphtalène ou pyridine condensé, par réaction d'halogénures 2-halo-benzyliques dans un solvant organique et en présence d'une substance basique avec du soufre élémentaire, caractérisé en ce que l'on fait réagir des halogénures 2-halo-benzyliques de la formule

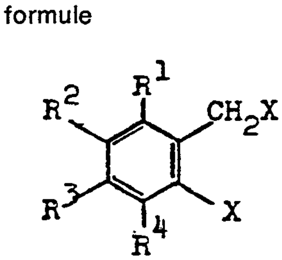

(II)

dans laquelle $R^1$, $R^2$, $R^3$ et $R^4$ possèdent la signification définie et les divers substituants X peuvent être identiques ou différents et désigner chacun un atome de chlore ou de brome, et du soufre dans un rapport de 2,5 à 3,5 atomes-grammes de soufre pour 1 mole du composé de départ II, à une température de 50 à 200 °C en présence d'éthers mono-alkyliques de glycols comme solvants et d'un alcanolate de métal alcalin ajouté sous forme d'une solution alcanolique comme substance basique, la proportion totale d'alcanol libre étant réglée pendant la réaction à un rapport de 0 à 30 % en poids d'alcanol par rapport à l'éther mono-alkylique de glycol.